(19) Europäisches Patentamt
European Patent Office
Office européen des brevets

(11) Veröffentlichungsnummer: **0 386 654 A1**

# (12) EUROPÄISCHE PATENTANMELDUNG

(21) Anmeldenummer: **90104087.3**

(22) Anmeldetag: **02.03.90**

(51) Int. Cl.[5]: **C07D 303/48, C07B 57/00, //A61K31/335**

(30) Priorität: **04.03.89 DE 3906953**

(43) Veröffentlichungstag der Anmeldung: **12.09.90 Patentblatt 90/37**

(84) Benannte Vertragsstaaten: **GR**

(71) Anmelder: **Byk Gulden Lomberg Chemische Fabrik GmbH**
**Byk-Gulden-Strasse 2**
**D-7750 Konstanz(DE)**

(72) Erfinder: **Kohl, Bernhard, Dr.**
**Heinrich-v.Tettingen-Strasse 35a**
**D-7750 Konstanz(DE)**
Erfinder: **Eistetter, Klaus, Dr.**
**Säntisblick 7**
**D-7750 Konstanz(DE)**
Erfinder: **Wolf, Horst P.O., Dr.**
**Im Tal 17**
**D-7753 Allensbach 4(DE)**

## (54) Verfahren zur Herstellung optisch reiner Oxirancarbonsäuren.

(57) Die Erfindung betrifft ein Verfahren zur Herstellung optisch reiner (R)-Oxirancarbonsäuren der Formel I,

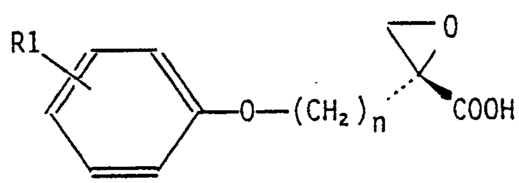

(I)

worin R1 und n die in der Beschreibung genannten Bedeutungen haben, mit (+)-Dehydroabietylamin als Spaltungsmittel.

EP 0 386 654 A1

## Verfahren zur Herstellung optisch reiner Oxirancarbonsäuren

### Anwendungsgebiet der Erfindung

Die Erfindung betrifft ein Verfahren zur Herstellung optisch reiner Oxirancarbonsäuren. Die erfindungsgemäß hergestellten Oxirancarbonsäuren werden in der pharmazeutischen Industrie zur Herstellung von Arzneimitteln eingesetzt.

### Bekannter technischer Hintergrund

In der Europäischen Patentanmeldung 0 046 590 werden Phen(alk)oxy-substituierte Oxirancarbonsäuren beschrieben, die bei der Synthese in Form von racemischen Gemischen anfallen. In der EP 0 046 590 wird außerdem beschrieben, daß diese racemischen Gemische mittels bekannter Verfahren in die Enantiomeren getrennt werden können, beispielsweise mit optisch aktiven Spaltungsmitteln, mit deren Hilfe man die Racemate in Diastereomere umwandelt, die anschließend durch selektive Kristallisation getrennt und in die entsprechenden optischen Isomeren übergeführt werden. Als optisch aktive Spaltungsmittel sind in der EP 0 046 590 beispielsweise optisch aktive Basen, wie 1- oder d-1-Phenylethylamin, Cinchonidin oder d-Ephedrin genannt.

### Beschreibung der Erfindung

Es wurde nun ein Verfahren gefunden, mit dessen Hilfe racemische Gemische chiraler Oxirancarbonsäuren überraschend glatt und in hoher Ausbeute in die optischen Antipoden getrennt werden können. Das Verfahren betrifft die Herstellung optisch reiner (R)-Oxirancarbonsäuren der Formel I,

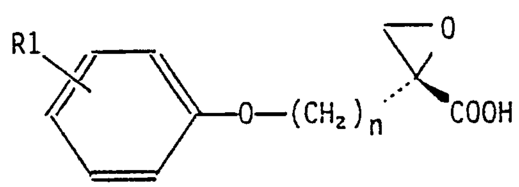

(I)

worin
R1 Wasserstoff, Chlor oder Fluor und
n die Zahlen 3, 4, 5, 6 oder 7 bedeutet,
und ihrer Salze,
aus den Racematen der Oxirancarbonsäuren durch Umsetzung mit optisch aktiven Basen, dadurch gekennzeichnet, daß als Base die Verbindung (+)-Dehydroabietylamin eingesetzt wird.

Als Salze seien alle Salze mit Basen genannt, beispielsweise Erdalkalisalze, bevorzugt Alkalisalze, insbesondere das Kaliumsalz. Besonders bevorzugt sind die verfahrensgemäß zunächst erhaltenen (+)-Dehydroabietylaminsalze von Verbindungen der Formel I, die ebenfalls Gegenstand der Erfindung sind.

Das Verfahren wird in geeigneten inerten, vorzugsweise polaren Lösungsmitteln durchgeführt. Als geeignete Lösungsmittel seien beispielsweise Essigester, Tetrahydrofuran, Aceton, n-Propanol, Isopropanol, Wasser oder vorzugsweise Dioxan oder Isobutylmethylketon genannt.

Bei der Umsetzung der Racemate der Oxirancarbonsäuren mit (+)-Dehydroabietylamin kann das Amin im Überschuß, in der genau berechneten stöchiometrischen Menge oder im Unterschuß eingesetzt werden. Vorteilhafterweise wird ein Unterschuß an (+)-Dehydroabietylamin (bevorzugt 0,55 bis 0,75 Moläquivalente) eingesetzt.

Die Trennung der nach der Umsetzung der Racemate der Oxirancarbonsäuren mit (+)-Dehydroabietylamin erhaltenen diastereomeren Salze erfolgt auf eine dem Fachmann vertraute Weise, bevorzugt durch fraktionierte Kristallisation.

Die Freisetzung der (R)-Oxirancarbonsäuren aus den diastereomeren Salzen erfolgt beispielsweise durch Umsetzung mit anorganischen Säuren, wie z.B. Schwefelsäure, oder mit Hilfe von sauren Ionenaustauschern. Vorzugsweise werden die (R)-Oxirancarbonsäuren über intermediär herzustellende Alkalisalze,

bevorzugt über die Kaliumsalze, und deren anschließende Umsetzung mit anorganischen Säuren erhalten. Die Alkalisalze werden hierbei durch Umsetzung der diastereomeren Dehydroabietylaminsalze mit Alkalilauge in organischen Lösungsmitteln oder Gemischen organischer Lösungsmittel, wie Dioxan, Dichlormethan, Ethanol, Methanol, Aceton oder bevorzugt Isobutylmethylketon hergestellt. Für die anschließende Freisetzung der Oxirancarbonsäuren werden anorganische Säuren wie Schwefelsäure oder Phosphorsäure eingesetzt.

Wenn nicht die (R)-Oxirancarbonsäuren selbst sondern ihre Alkylester die gewünschten Produkte sind, so können diese auch direkt aus den Alkalisalzen durch Umsetzung mit geeigneten Alkylierungsmitteln (z.B. mit Dialkylsulfaten) in für Alkylierungsreaktionen üblichen Lösungsmitteln (vorzugsweise in N-Methylpyrrolidon) bei Temperaturen zwischen 20 und 100°C, vorzugsweise zwischen 50 und 80°C, hergestellt werden.

Durch das erfindungsgemäße Verfahren bevorzugt zu trennende racemische Oxirancarbonsäuren sind solche der Formel I, in denen n die Zahlen 5 oder 6 bedeutet.

Besonders bevorzugte, durch das erfindungsgemäße Verfahren herstellbare Oxirancarbonsäuren sind:
(R)-(+)-2-[6-(4-Chlorphenoxy)-hexyl]-oxiran-2-carbonsäure
(R)-(+)-2-[6-(4-Fluorphenoxy)-hexyl]-oxiran-2-carbonsäure
(R)-2-(6-Phenoxyhexyl)-oxiran-2-carbonsäure
(R)-2-[5-(4-Chlorphenoxy)-pentyl]-oxiran-2-carbonsäure
(R)-2-[5-(4-Fluorphenoxy)-pentyl]-oxiran-2-carbonsäure
(R)-2-(5-Phenoxypentyl)-oxiran-2-carbonsäure

Die folgenden Beispiele sollen die Erfindung näher erläutern, ohne sie einzuschränken. F. steht für Schmelzpunkt, h für Stunde(n).

## Beispiele

### 1. (R)-(+)-2-[6-(4-Chlorphenoxy)-hexyl]-oxiran-2-carbonsäure

a) (R)-(+)-2-[6-(4-Chlorphenoxy)-hexyl]-oxiran-2-carbonsäure-(+)-dehydroabietylaminsalz.

Zu 300 g (±)-2-[6-(4-Chlorphenoxy)-hexyl]-oxiran-2-carbonsäure, gelöst in 8 l Dioxan, läßt man 302 g (+)-Dehydroabietylamin {$[\alpha]_D^{20}$ = + 60 bis + 63° (c = 2,4; Pyridin)}, gelöst in 4 l Dioxan, bei Raumtemperatur zutropfen. Man rührt 20 h bei 20°C, filtriert vom auskristallisierten Feststoff und wäscht mit 2 x 500 ml Dioxan. Das Rohkristallisat wird aus 8 l Dioxan umkristallisiert, wobei man zur vollständigen Kristallisation erneut 20 h bei 20°C rührt. Absaugen über eine Filternutsche und Trocknen bei 40°C im Vakuum liefert 220 g (R)-(+)-2-[6-(4-Chlorphenoxy)-hexyl]-oxiran-2-carbonsäure-(+)-dehydroabietylaminsalz vom F. 145 - 147°C (farbloses Kristallisat); $[\alpha]_D^{22}$ = + 27,9° (c = 1; Methanol).

Alternativ läßt sich das (+)-Dehydroabietylaminsalz auch folgendermaßen herstellen:
Zu einer Lösung von 1,020 kg (±)-2-[6-(4-Chlorphenoxy)hexyl]-oxiran-2-carbonsäure in 24 l Isobutylmethylketon tropft man 654 g (0,65 Äquivalente) (+)-Dehydroabietylamin, gelöst in 4 l Isobutylmethylketon innerhalb von 3 h zu. Nach 48 h wird vom ausgefallenen Kristallisat über ein Druckfilter filtriert, das Produkt einmal in 7 l Isobutylmethylketon aufgerührt, einmal filtriert und getrocknet (40°C / 10mbar). Man erhält 860 g (88 %) (R)-(+)-2-[6-(4-Chlorphenoxy)-hexyl]-oxiran-2-carbonsäure-(+)-dehydroabietylaminsalz vom F. 147-149°C; $[\alpha]_D^{22}$ = + 27,9° (c = 1; Methanol).

b) Kalium-(R)-(+)-2-[6-(4-chlorphenoxy)-hexyl]-oxiran-2-carboxylat

197 g (R)-(+)-2-[6-(4-Chlorphenoxy)-hexyl]-oxiran-2-carbonsäure-(+)-dehydroabietylaminsalz werden in 3,6 l Dichlormethan und 400 ml Methanol gelöst. Innerhalb von 1 h wird 1 Äquivalent Kaliumhydroxid in 200 ml Methanol zugetropft. Man rührt noch 1 h, filtriert vom gebildeten Feststoff und wäscht zweimal mit je 200 ml Dichlormethan. Man erhält 107 g (94 %) Kalium-(R)-(+)-2-[6-(4-Chlorphenoxy)-hexyl]-oxiran-2-carboxylat als farbloses Pulver vom F. 198°C (Zersetzung). $[\alpha]_D^{22}$ = + 19,0° (c = 1; Methanol).

c) (R)-(+)-2-[6-(4-Chlorphenoxy)-hexyl]-oxiran-2-carbonsäure

10 g Kalium-(R)-(+)-2-[6-(4-chlorphenoxy)-hexyl]-oxiran-2-carboxylat werden in 100 ml Dichlormethan und 100 ml Wasser suspendiert. 2N Schwefelsäure wird zugetropft bis pH 3,5 erreicht ist. Nach Phasentrennung und Extraktion der Wasserphase mit 30 ml Dichlormethan werden die vereinigten organischen Phasen zweimal mit je 50 ml Schwefelsäure (pH 3,5) gewaschen. Anschließend engt man die organische Phase im Vakuum ein und kristallisiert die Titelverbindung aus Petrolether (50/70). Man erhält 8,5 g (96 %) der Titelverbindung (R)-(+)-2-[6-(4-Chlorphenoxy)-hexyl]-oxiran-2-carbonsäure in Form farbloser Kristalle vom F. 68°C. $[\alpha]_D^{22}$ = + 12,9° (c = 1; Methanol).

Mittels $^1$H-NMR-Analytik mit einem Äquivalent Cinchonidin als Shiftreagens läßt sich die Enantiomerenreinheit der nach 1a) - 1c) hergestellten Titelverbindung zu > 99 % (R)-(+)-Enantiomer bestimmen.

d) (R)-(+)-2-[6-(4-Chlorphenoxy)-hexyl]-oxiran-2-carbonsäure

Alternativ erhält man die Titelverbindung wie folgt: 1 g (R)-(+)-2-[6-(4-Chlorphenoxy)-hexyl]-oxiran-2-carbonsäure-(+)-dehydroabietylaminsalz, gelöst in 50 ml Dichlormethan und 20 ml Wasser, wird mit 1,5 Äquivalenten 2N Schwefelsäure versetzt. Das ausgefallene Salz [(+)-Dehydroabietylaminsulfat] wird abfiltriert, die organische Phase des Filtrates abgetrennt, noch zweimal mit Wasser extrahiert, das Dichlormethan am Rotavapor im Vakuum entfernt und durch Zugabe von Petrolether (50/70) die Titelverbindung kristallisiert. Man erhält 0,71 g (R)-(+)-2-[6-(4-Chlorphenoxy)-hexyl]-oxiran-2-carbonsäure vom F. 65 - 66°C $[\alpha]_D^{22}$ = 12,9° (c = 1; Methanol).

2. (R)-(+)-2-(6-Phenoxyhexyl)-oxiran-2-carbonsäure

Nach der in Beispiel 1 a) angegebenen Arbeitsweise erhält man aus 4 g (±)-2-(6-Phenoxyhexyl)-oxiran-2-carbonsäure durch Umsetzung mit 3,5 g (+)-Dehydroabietylamin in 280 ml Dioxan nach zweimaliger Umkristallisation des ausgefallenen Rohsalzes aus jeweils 150 ml Dioxan 2,5 g (R)-(+)-2-(6-Phenoxyhexyl)-oxiran-2-carbonsäure-(+)-dehydroabietylaminsalz. Dessen Überführung nach der in 1 b) angegebenen Arbeitsweise zum Kalium-(R)-(+)-2-(6-Phenoxyhexyl)-oxiran-2-carboxylat und die anschließende Freisetzung der Titelverbindung nach der in Beispiel 1 c) angegebenen Arbeitsweise liefert 1,1 g (55 %) (R)-(+)-2-(6-Phenoxyhexyl)-oxiran-2-carbonsäure als farbloses Kristallisat.

3. (R)-(+)-2-[5-(4-Fluorphenoxy)pentyl]-oxiran-2-carbonsäure

Nach der in Beispiel 1 a) angegebenen Arbeitsweise erhält man aus 0,5 g (±)-2-[5-(4-Fluorphenoxy)-pentyl]-oxiran-2-carbonsäure durch Umsetzung mit 0,56 g (+)-Dehydroabietylamin in 15 ml Dioxan 0,32 g (62 %) (R)-(+)-2-[5-(4-Fluorphenoxy)pentyl]-oxiran-2-carbonsäure-(+)-dehydroabietylaminsalz $[\alpha]_D^{22}$ = + 30,5° (c = 1; Methanol). Dessen Überführung nach der in 1 b) angegebenen Arbeitsweise zum Kaliumsalz und die anschließende Freisetzung der Titelverbindung nach der in Beispiel 1 c) angegebenen Arbeitsweise liefert die Titelverbindung vom F. 55 - 56°C; $[\alpha]_D^{22}$ = + 12,9° (c = 1; Methanol).

## Gewerbliche Anwendbarkeit

Die optisch reinen Oxirancarbonsäuren und ihre Salze sind pharmakologisch hochwirksame, interessante Verbindungen, deren Einsatz in der Humanmedizin (z.B. zur Behandlung von Glucose- und Fettstoffwechselstörungen) diskutiert wird. Da die pharmakologische Wirksamkeit insbesondere an die bei den Verbindungen der Formel I dargestellte (R)-Form sowie an die Ester dieser Verbindungen geknüpft ist [siehe z.B. Drugs of the future 11, 1034 (1986)], bestand ein erheblicher Bedarf an einem Verfahren, mit dessen Hilfe die chiralen Oxirancarbonsäuren glatt und in hoher Ausbeute in ihre optischen Antipoden aufgetrennt werden können, wobei es von besonderer Bedeutung war, das wirksame (R)-Enantiomer in reiner Form zu erhalten. Die in der EP 0 046 590 offenbarten Hinweise zur Trennung der Racemate in ihre Enantiomeren stellen nur eine allgemeine Arbeitsanleitung für den Fachmann dar, bei deren Einhaltung er zwar zum gewünschten Ergebnis gelangt, jedoch nur mit geringeren Ausbeuten oder mit aufwendigeren Trennoperationen als im erfindungsgemäßen Verfahren.

Die Eignung von (+)-Dehydroabietylamin zur Verwendung als basisches Spaltungsmittel, die mit sehr guter Ausbeute zu einer Auftrennung der Oxirancarbonsäure-Racemate und zu den (R)-Enantiomeren mit

hoher optischer Reinheit führt, überrascht ganz besonders, da es sich bei (+)-Dehydroabietylamin um ein primäres Amin handelt. Bei der Umsetzung dieses primären Amins mit den Oxirancarbonsäuren I hätte man in erster Linie die Addition an eine Oxiranbindung unter Ringöffnung erwartet, eine Reaktion, wie sie für die genannten Edukte als klassische Standardreaktion beschrieben ist.

**Ansprüche**

1. Verfahren zur Herstellung optisch reiner (R)-Oxirancarbonsäuren der Formel I,

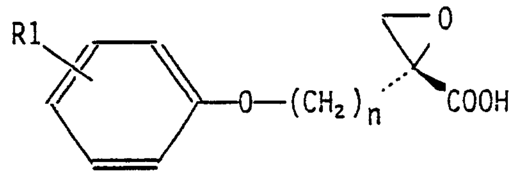

(I)

worin
R1 Wasserstoff, Chlor oder Fluor und
n die Zahlen 3, 4, 5, 6 oder 7 bedeutet,
und ihrer Salze,
aus ihren Racematen durch Umsetzung der Racemate mit optisch aktiven Basen, Trennung der diastereomeren Salze und anschließende Überführung in ihre Alkalisalze und/oder Freisetzung der Säuren daraus, dadurch gekennzeichnet, daß als optisch aktive Base (+)-Dehydroabietylamin verwendet wird.

2. Verfahren nach Anspruch 1, wobei vor der Freisetzung der Säuren die diastereomeren Dehydroabietylaminsalze in die entsprechenden Alkalisalze überführt werden.

3. Verfahren nach Anspruch 1 zur Herstellung optisch reiner Oxirancarbonsäuren der Formel I nach Anspruch 1, und ihrer Salze, wobei R1 Wasserstoff, Chlor oder Fluor und n die Zahlen 5 oder 6 bedeutet.

4. Verfahren nach Anspruch 1 zur Herstellung optisch reiner Oxirancarbonsäuren der Formel I nach Anspruch 1, und ihrer Salze, dadurch gekennzeichnet, daß eine Verbindung ausgewählt aus der Gruppe bestehend aus
(R)-(+)-2-[6-(4-Chlorphenoxy)-hexyl]-oxiran-2-carbonsäure
(R)-(+)-2-[6-(4-Fluorphenoxy)-hexyl]-oxiran-2-carbonsäure
(R)-2-(6-Phenoxyhexyl)-oxiran-2-carbonsäure
(R)-2-[5-(4-Chlorphenoxy)-phenyl]-oxiran-2-carbonsäure
(R)-2-[5-(4-Fluorphenoxy)-phenyl]-oxiran-2-carbonsäure und
(R)-2-(5-Phenoxypentyl)-oxiran-2-carbonsäure
oder ein Salz davon hergestellt wird.

5. (R)-Oxirancarbonsäure-(+)-dehydroabietylaminsalze der Formel II,

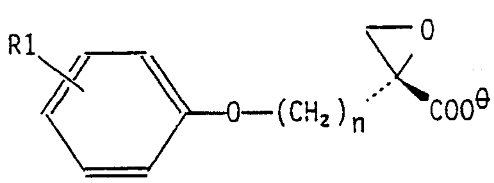

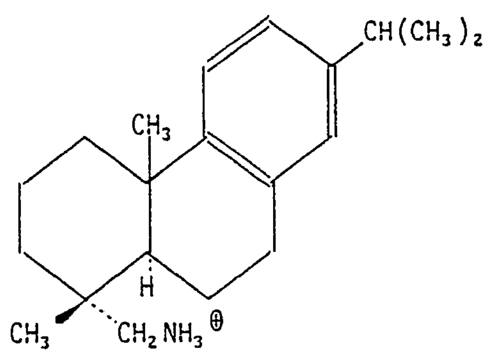

(II)

worin
R1 Wasserstoff, Chlor oder Fluor und
n die Zahlen 3, 4, 5, 6 oder 7 bedeutet.

6. (R)-(+)-2-[6-(4-Chlorphenoxy)-hexyl]-oxiran-2-carbonsäure-(+)-dehydroabietylaminsalz.

Europäisches Patentamt

# EUROPÄISCHER RECHERCHENBERICHT

Nummer der Anmeldung

EP 90 10 4087

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,Y | EP-A-0 046 590 (BYK GULDEN LOMBERG)<br>* Insgesamt, insbesondere Seite 14, Zeilen 11-28 *<br>--- | 1-6 | C 07 D 303/48<br>C 07 B 57/00 //<br>A 61 K 31/335 |
| Y | DE-A-1 805 681 (MERCK & CO.)<br>* Insgesamt, inbesondere Seite 17, Beispiel 12 *<br>----- | 1-6 | |
| | | | **RECHERCHIERTE SACHGEBIETE (Int. Cl.5)** |
| | | | C 07 D 303/00<br>C 07 B 57/00 |

Der vorliegende Recherchenbericht wurde für alle Patentansprüche erstellt

| Recherchenort | Abschlußdatum der Recherche | Prüfer |
|---|---|---|
| DEN HAAG | 25-05-1990 | ALLARD M.S. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)